# EUROPÄISCHE PATENTSCHRIFT

(11) **EP 0 793 647 B1**
(45) Veröffentlichungstag und Bekanntmachung des Hinweises auf die Patenterteilung: **15.12.1999**
(21) Anmeldenummer: 95932758.6
(22) Anmeldetag: 15.09.1995
(51) Int. Cl.: C07C 305/10, B01F 17/00, C11D 1/08, C11D 1/29, C11D 1/34, C11D 1/88

(54) **AMPHIPHILE VERBINDUNGEN MIT MINDESTENS ZWEI HYDROPHILEN UND MINDESTENS ZWEI HYDROPHOBEN GRUPPEN AUF DER BASIS VON DI-, OLIGO- ODER POLYOLETHERN**
AMPHIPHILIC COMPOUNDS WITH AT LEAST TWO HYDROPHILIC AND AT LEAST TWO HYDROPHOBIC GROUPS BASED ON DI, OLIGO OR POLYOLETHERS
COMPOSES AMPHIPHILES COMPORTANT AU MOINS DEUX GROUPES HYDROPHILES ET AU MOINS DEUX GROUPES HYDROPHOBES A BASE DE DI, OLIGO OU POLYOLETHERS

(30) Priorität: 21.11.1994 DE 4441363
(43) Veröffentlichungstag der Anmeldung: 10.09.1997
(73) Patentinhaber: RWE-DEA AKTIENGESELLSCHAFT FÜR MINERALOEL UND CHEMIE, 22297 Hamburg (DE)
(72) Erfinder: KWETKAT, Klaus, D-59192 Bergkamen (DE); SCHRÖDER, Wolfgang, D-46282 Dorsten (DE)
(74) Vertreter: Schupfner, Gerhard D., Dr. Dipl.-Chem.
(86) Internationale Anmeldenummer: EP9503634
(87) Internationale Veröffentlichungsnummer: WO9616033

(56) Entgegenhaltungen:
- DE-A- 3 843 327
- US-A- 2 091 956
- US-A- 5 160 450

## Beschreibung

Die Erfindung betrifft amphiphile Verbindungen mit mindestens zwei hydrophilen und mindestens zwei hydrophoben Gruppen auf der Basis von Di-, Oligo- oder Polyolethern.

Als amphiphile Substanzen sind eine große Vielfalt an anionischen, kationischen, nichtionischen und zwitterionischen Verbindungen bekannt. Die weitaus meisten dieser Substanzen bestehen aus einer hydrophilen Kopfgruppe und wenigstens einem hydrophoben Teil.

Bei den amphiphilen Substanzen besteht aus ökologischen Gründen, z. B. bezüglich der Verringerung des Verpackungs- und Transportaufwandes die Notwendigkeit, immer größere Wirkung pro Masse an eingesetzter Substanz zu erzielen. Da eine Optimierung durch Mischung von amphiphilen Substanzen nur sehr begrenzt weiterführt, sind neue amphiphile Substanzen mit einem höheren Wirkungsgrad erforderlich. Es müssen daher insbesondere Stoffe mit niedrigeren kritischen Micellbildungskonzentrationen und/oder niedrigeren Ober- und Grenzflächenspannungen gefunden werden, um die Einsatzmengen an Wirksubstanz deutlich reduzieren zu können.

Erste Lösungsansätze in dieser Richtung durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G. Beinert, P. Marie, R. Zana, Langmuir, 9 (1993) 1465].

Anionische grenzflächenaktive Verbindungen mit wenigstens zwei hydrophilen und wenigstens zwei hydrophoben Gruppen sind bisher nur auf der Basis von Diglycidylethern hergestellt worden (US 5 160 450, JP 01 304 033, JP 4 124 165). Diglycidylether gelten jedoch als toxikologisch bedenklich und sind recht teuer. Darüber hinaus wird für ihre Herstellung Epichlorhydrin verwendet, was zu großen Mengen an Reststoffen führt, so daß diese Verbindungen unter ökotoxikologischen wie auch ökonomischen Gesichtspunkten nicht mehr zeitgemäß sind.

Es bestand daher die Aufgabe, amphiphile Verbindungen aufzufinden, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne großen Anfall von unerwünschten Nebenprodukten hergestellt werden können.

Die Aufgabe wird erfindungsgemäß durch amphiphile Di-, Oligo- oder Polyether, deren Grundkörper aus Di-, Oligo- oder Polyolethern und α-Epoxiden hergestellt werden können, gelöst. Die entsprechenden Di-, Oligo- oder Polyether können alkoxyliert und anschließend oder direkt in anionische, amphiphile Verbindungen, überführt werden, indem man z. B. die vorgenannten Verbindungen mit Schwefeltrioxid/Inertgas, Oleum, Chlorsulfonsäure oder Amidosulfonsäure, mit Polyphosphorsäure, mit einer Halogenessigsäure, mit einem Sulton, mit einem Taurin oder mit Maleinsäureanhydrid und Natriumhydrogensulfit umsetzt und jeweils anschließend neutralisiert.

Bei den erfindungsgemäßen amphiphilen Verbindungen handelt es sich daher um Verbindungen der allgemeinen Formeln Ia, Ib, Ic, Id und deren Mischungen 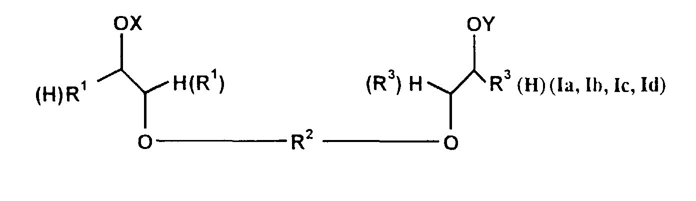 wobei R¹, R², R³, X und Y in der Formel I die im folgenden beschriebenen Bedeutungen haben und wobei R¹ und R³ in den Formeln Ia, Ib, Ic, Id je einmal vorkommen.

R¹ und R³ stehen unabhängig voneinander für einen unverzweigten oder verzweigten, gesättigten oder ungesättigten Kohlenwasserstoffrest mit 1 bis 22, vorzugsweise 6 bis 18, Kohlenstoffatomen.

Es seien als Substituenten R¹ und R³ im einzelnen die Reste Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Henicosyl, n-Docosyl und ihre verzweigtkettigen Isomeren sowie die entsprechenden einfach, zweifach oder dreifach ungesättigten Alkene genannt.

R² bedeutet einen Spacer, bestehend aus einer unverzweigten oder verzweigten Kette mit 2 bis 100 Kohlenstoffatomen, die 0 bis 20 Sauerstoff-, 0 bis 20 Stickstoff-, 0 bis 4 Schwefel- und 0 bis 3 Phosphoratome enthält und die 0 bis 20 funktionelle Seitengruppen, wie z. B. Hydroxyl-, Carbonyl-, Carboxyl-, Amino- und/oder Acylaminogruppen, aufweist.

Der Spacer R² bedeutet insbesondere
◆ als Grundkörper unverzweigte oder verzweigte Alkylenketten

   -CₐH₂ₐ- (II)

   mit a = 2 bis 18, vorzugsweise a = 2 bis 6;
◆ als Grundkörper unverzweigte oder verzweigte Alkenylenketten

   -C_{b}H_{2b}-CH=CH-C_{c}H_{2c}- (III)

   mit b ◆ c = 2 bis 16, wobei b und c jeweils größer als Null sind;
◆ als Grundkörper unverzweigte oder verzweigte Alkinylenketten

   -C_{d}H_{2d}-C≡C-CₑH₂ₑ- (IV)

   mit d + e = 2 bis 16, wobei d und e jeweils größer als Null sind, und wobei bei den Grundkörpern gemäß den Formeln II bis IV der Spacer an einer beliebigen Stelle der Kette unabhängig voneinander 0 bis 4 Carbonyl-, Carboxyl-, Amino- oder Acylaminogruppen enthält;
◆ Alicyclen gemäß der Formel V

   -C_{f}H_{2f}-cyclo C₆H₁₀-C_{g}H_{2g}- (V)

   mit f und g gleich unabhängig voneinander je 1 bis 6
   oder gemäß der Formel VI

   -3(4),8(9)-di(methylen)-tricyclo[5.2.1.0^{2.6}]decan- (VI);
◆ gegebenenfalls substituierte Aromaten gemäß der Formel VII

   -CₕH₂ₕ-C₆R₄-(CᵢH₂ᵢ-C₆R₄)_{j₁}-C_{j₂}H_{2j₂}- (VII)

   oder gemäß der Formel VIII

   -CₕH₂ₕ-C₁₀R₆-CⱼH₂ⱼ- (VIII)

   mit h, j, j₁ und j₂ gleich unabhängig voneinander je 0 bis 8 und i = 0 bis 8 und mit R gleich unabhängig voneinander jeweils H oder C₁- bis C₆-Alkyl;
◆ eine Kette mit funktionellen Seitengruppen, insbesondere Hydroxyl-, Carbonyl-, Carboxyl-, Amino- und/oder Acylaminogruppen.

Weiterhin enthält der Spacer R² je 0 bis 20, vorzugsweise 1 bis 12, Sauerstoff- und/oder Stickstoffatome, 0 bis 4 Schwefel- und 0 bis 3 Phosphoratome, wobei mindestens eines der Heteroatome mindestens einmal vorkommt.

R² hat damit weiterhin insbesondere die Bedeutung
◆ einer Verbindung gemäß der Formel IX

   -CₖH₂ₖ-CₓR_{y}-Z-CₓR_{y}-CₗH₂ₗ- (IX)

   mit k und l gleich unabhängig voneinander je 0 bis 8, x = 6 und y = 4 oder x = 10 und y = 6 oder x = 14 und y = 8, und Z = 0, CO, NH, N-CH₂-CH(OX)-R¹, NR¹, N-C(O)R¹, SO₂
   oder gemäß der Formel IXa

   -CH₂-CH(OCH₂CH(OX)-R¹)-CH₂- oder ein Isomer (IXa)

   oder 2,2'-Methylen-bis-(1,3-dioxolan-5-methylen)-oder Acetale, insbesondere Diacetale aus Dialdehyden und Di-, Oligo- oder Polyolen,
   wobei R¹ einen Kohlenwasserstoffrest mit 1 bis 22 Kohlenstoffatomen bedeutet und X für einen Substituenten steht, der eine funktionelle Gruppe trägt und die weiter unten angegebene Bedeutung hat,
◆ einer Verbindung gemäß der Formel X

   -CₘH₂ₘ-(OCₙH₂ₙ)ₚ-C_{q}H_{2q}- (X)

   mit m = 1 bis 4, n = 2 bis 4, p = 1 bis 20, vorzugsweise p = 1 bis 4, und q = 0 bis 4,
   wobei auch gemischte Alkoxideinheiten auftreten können und dann die Reihenfolge der Alkoxideinheiten beliebig ist,
◆ einer Verbindung gemäß der Formel XI

   -CᵣH₂ᵣ(RNCₛH₂ₛ)ₜ-CᵤH₂ᵤ- (XI)

   oder gemäß der Formel XII

   -[CᵣH₂ᵣ[RN-C(O)-NR]ₜ-CᵤH₂ᵤ]_{w}- (XII)

   oder gemäß der Formel XIII

   -[CᵣH₂ᵣ[RNC(O)CᵥH₂ᵥC(O)NR]ₜ-CᵤH₂ᵤ]_{w}- (XIII)

   oder gemäß der Formel XIV

   -[CᵣH₂ᵣ[RN-C(O)-CH=CH-C(O)-NR]ₜ-CᵤH₂ᵤ]_{w}- (XIV)

   oder gemäß der Formel XV

   -[CᵣH₂ᵣ[RNC(O)CₓR_{y}C(O)NR]ₜ-CᵤH₂ᵤ]_{w}- (XV)

   mit r = 2 bis 4, s = 2 bis 4, t = 1 bis 20, vorzugsweise t = 1 bis 4,
   u = 2 bis 4, v = 0 bis 12, w = 1 bis 6, x = 6 und y = 4 oder
   x = 10 und y = 6 oder
   x = 14 und y = 8
   mit R gleich unabhängig voneinander H oder C₁- bis C₆-Alkyl.

X und Y stehen unabhängig voneinander für Substituenten der Formel XVI

-(C₂H₄O)_{α}(C₃H₆O)_{β}H (XVI)

mit α = 0 bis 50, vorzugsweise α = 10 bis 30,
β = 0 bis 60, vorzugsweise β = 20 bis 40,
und α + β = 1 bis 100, vorzugsweise α + β = 10 bis 50,
und wobei die Alkoxideinheiten statistisch oder blockweise eingebunden sind und die Reihenfolge beliebig ist
oder für Substituenten der Formel XVII

-(C₂H₄O)_{γ}(C₃H₆O)_{δ}-FR (XVII)

mit jeweils γ = 0 bis 20, vorzugsweise γ = 0 bis 8,
δ = 0 bis 20, vorzugsweise δ = 0 bis 12,
und γ + δ = 0 bis 40, vorzugsweise γ + δ = 5 bis 20,
wobei FR für einen funktionellen Rest -CH₂-COOM, -SO₃M, -P(O)(OM)₂, -C₂H₄-SO₃M oder

-O-C(O)-C₂H₃(SO₃M)-CO₂M'

mit M, M' = Alkali, Ammonium-, Alkanolammonium oder ½ Erdalkali, steht
oder für Substituenten der Formel XVIII

-CH₂[CHO(C₂H₄O)_{ε}(C₃H₆O)_{η}H]_{z}-CH₂-O(C₂H₄O)_{µ}(C₃H₆O)_{ρ}-H (XVIII)

mit z = 3 bis 6, vorzugsweise z = 4
und ε bzw. µ = 0 bis 30, vorzugsweise ε bzw. µ = 0 bis 10,
und η bzw. ρ = 0 bis 30, vorzugsweise η bzw. ρ = 0 bis 10
und wobei die Alkoxideinheiten ebenfalls statistisch oder blockweise eingebunden sind und die Reihenfolge beliebig ist.

Der Alkoxylierungsgrad ist jeweils ein Mittelwert und kann jeden beliebigen, auch nicht ganzzahligen Wert in den angegebenen Grenzen einnehmen.

Die erfindungsgemäßen amphiphilen Verbindungen zeichnen sich meist durch extrem niedrige kritische Micellbildungskonzentrationen (CMC) und sehr niedrige Oberflächen- und Grenzflächenspannungen (z. B. gegen Paraffin) aus, was auf ihre besondere Struktur - wenigstens zwei hydrophile Gruppen und wenigstens zwei hydrophobe Gruppen - zurückgeführt werden muß. Darüber hinaus weisen die meisten von ihnen ein recht hohes hydrophiles Suspendiervermögen auf, das etwa auf halbem Wege zwischen dem konventioneller Tenside und dem des Pentanatriumtripolyphosphats liegt. Einige dieser Verbindungen sind extrem schnelle Netzmittel.

Die amphiphilen Verbindungen gemäß dieser Erfindung eignen sich insbesondere als Emulgatoren, Demulgatoren, Detergenzien, Dispergatoren und Hydrotropica in Industrie und Haushalt, beispielsweise auf den Gebieten Metallbearbeitung, Erzgewinnung, Oberflächenveredelung, Waschen und Reinigen, Kosmetik, Medizin und Nahrungsmittelverarbeitung und -zubereitung.

Hierbei können sie mit allen gängigen anionischen, nichtionischen, kationischen und ampholytischen grenzflächenaktiven Substanzen kombiniert werden. Als Beispiele für nichtionische grenzflächenaktive Substanzen, die für eine Kombination eingesetzt werden können, seien Fettsäureglyceride, Fettsäurepolyglyceride, Fettsäureester, Ethoxylate höherer Alkohole, Polyoxyethylenfettsäureglyceride, Polyoxyethylenpropylenglykolfettsäureester, Polyoxyethylensorbitanfettsäureester, Polyoxyethylen-Rhizinusöl- oder gehärtete Rhizinusöl-Derivate, Polyoxyethylenlanolinderivate, Polyoxyethylenfettsäureamide, Polyoxyethylenalkylamine, Alkanolamine, Alkyl-aminoxide, Derivate von Eiweißhydrolysaten, Hydroxymischether, Alkylpolyglycoside und Alkylglucamide genannt.

Als Beispiele für anionische grenzflächenaktive Substanzen, die für Kombinationen eingesetzt werden können, seien Seifen, Ethercarbonsäuren und deren Salze, Alkylsulfonate, α-0lefinsulfonate, Sulfonate höherer Fettsäureester, höhere Alkoholsulfate, Alkoholethersulfate, Hydroxymischethersulfate, Salze von Phosphatestern, Tauride, Isethionate, lineare Alkylbenzolsulfonate, Cumolsulfonat, Alkylarylsulfonate, Sulfate der Polyoxyethylenfettsäureamide und Salze von Acylaminosäuren genannt.

Als Beispiele für kationische gängige grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Alkylpyridiniumsalze, Alkylisochinoliniumsalze, Benzethoniumchloride und kationische Acylaminosäurederivate genannt.

Als Beispiele für ampholytische grenzflächenaktive Substanzen, die für Kombinationen eingesetzt werden können, seien Aminosäuren, Betaine, Sulfobetaine, Imidazolinderivate, Sojaöllipide und Lecithin genannt.

Darüber hinaus können die erfindungsgemäßen amphiphilen Verbindungen auch für sich miteinander kombiniert werden.

Den erfindungsgemäßen amphiphilen Verbindungen können ebenfalls gängige Additive zugesetzt werden. Solche Additive werden speziell für eine Formulierung ausgewählt und umfassen üblicherweise anorganische Salze, wie Natriumchlorid und -sulfat, sowie Builder, Hydrotropica, UV-Absorber, Weichmacher, Chelatbildner, Viskositätsmodifizierer und Riechstoffe.

Die obengenannten Verbindungen lassen sich aus Di-, Oligo- oder Polyolen und mindestens der doppelten Anzahl von Äquivalenten an α-Epoxiden in Gegenwart eines Katalysators herstellen. Dabei kann der Katalysator aus der Gruppe basischer oder saurer Substanzen wie Alkalialkoholate, Alumosilikate, Schichtsilikate, Aluminiumoxide, Bortrifluoridetherat, Aluminiumchlorid, Niobsäure, HY-Zeolith und Alkalimetalle stammen. Die Reaktion läuft nicht mit einer vollständigen Selektivität für die Öffnung des Epoxides von der unsubstituierten Seite her, so daß Mischungen von primären und sekundären Di-, Oligo- oder Polyolen als Zwischenprodukte gebildet werden. Die flüssigen oder bei niedrigen Temperaturen erweichenden Produkte oder deren Alkoxylate können anschließend mit Schwefeltrioxid/Inertgas, Oleum, Chlorsulfonsäure oder Amidosulfonsäure, mit Polyphosphorsäure, mit einer Halogenessigsäure, mit einem Sulton mit einem Taurin oder mit Maleinsäureanhydrid und Natriumhydrogensulfit umgesetzt und mit wäßrigen Alkali- oder Erdalkalihydroxiden oder wäßrigem Ammoniak oder Alkanolaminen neutralisiert werden. Bei Bedarf werden die Produkte in wäßriger Lösung mit Wasserstoffperoxid (0,1 bis 2,0 %, bezogen auf Feststoff) gebleicht.

### Beispiele

Folgende Beispiele sollen die Erfindung erläutern, sie jedoch nicht darauf einschränken.

### Beispiel 1:

### R¹ = R³ = C₆H₁₃ R² = C₂H₄ X = Y = SO₃Na

In einem 250 ml-Dreihalskolben mit Rührer, Thermometer, Schutzgasanschluß und Rückflußkühler werden 53,1 g Ethylenglykol und 1,0 Gew.-% (bezogen auf Epoxid) Natriummethanolat vorgelegt, das Gemisch bis zur Rührfähigkeit erwärmt und 229,8 g 1,2-Epoxyoctan zugegeben. Das Reaktionsgemisch wird dann auf 180 °C erwärmt und die Reaktion nach 6 Stunden (Epoxid laut GC vollständig umgesetzt) beendet. Es wird mit Natriumhydrogencarbonatlösung gewaschen und das Produktisomerengemisch bei 130 bis 145 °C (0,1 mbar) destilliert. Ausbeute: 149,4 g (80 %), Reinheit laut GC > 95 %.

### Sulfatierung:

Zu einem Gemisch aus 61,3 g Chlorsulfonsäure und 31,8 g Essigsäure werden 49,6 g des oben erhaltenen Diols in 200 ml Dichlormethan so zugetropft, daß die Temperatur 5 °C nicht übersteigt. Anschließend wird bei Raumtemperatur 3 Stunden gerührt (laut Kontrolle per Dünnschichtchromatographie ist das Diolgemisch vollständig umgesetzt). Es wird mit 2-normaler Natriumcarbonatlösung neutralisiert und mit gesättigter Natriumhydrogencarbonatlösung verdünnt. Mit n-Butanol wird das Produkt extrahiert und anschließend der Alkohol entfernt. Die Reinheitskontrolle erfolgt per Dünnschichtchromatographie und NMR. Ausbeute: 70,3 g (80 % d. Th.), Reinheit: 90 % (Isomerengemisch). Charakteristische ¹³C-NMR Daten (DMSO-D₆):
76,3 ppm, 72,1 ppm, 73,0 ppm, 71,6 ppm, 69,9 ppm, 69,4 ppm, 30,6 ppm, 28,6 ppm, 28,4 ppm, 25,0 ppm, 21,4 ppm , 20,3 ppm, 20,2 ppm, 13,3 ppm. CMC (in VE-Wasser, 20 °C): 5,5 g/l, γ_{CMC} : 30,5 mN/m (VE-Wasser, 20 °C).

### Beispiel 2:

### R¹ = R³ = C₁₀H₂₁ R² = -C₂H₄-O-C₂H₄- X = Y = SO₃Na

In einem 250 ml-Dreihalskolben mit Rührer, Thermometer, Schutzgasanschluß und Rückflußkühler werden 21,4 g Diethylenglykol und 3,0 Gew.-% (bezogen auf Epoxid) Al₂O₃ vorgelegt, das Gemisch bis zur Rührfähigkeit erwärmt und 82,4 g 1,2-Epoxydodekan zugegeben. Das Reaktionsgemisch wird dann auf 180 °C erwärmt und die Reaktion nach 8 Stunden (Epoxid laut GC vollständig umgesetzt) beendet. Es wird mit Natriumhydrogencarbonatlösung gewaschen. Ausbeute: 61,9 g (52 %), Reinheit laut GC > 95 %, Isomerenverhältnis: sekundäres Diol zu primärem Diol (R¹ und OX bzw. R³ und OY teilweise vertauscht): 80 : 20.

### Sulfatierung:

Zu einem Gemisch aus 47,1 g Chlorsulfonsäure und 24,1 g Essigsäure werden 49,6 g des oben erhaltenen Diols in 200 ml Dichlormethan so zugetropft, daß die Temperatur 5 °C nicht übersteigt. Anschließend wird bei Raumtemperatur 3 Stunden gerührt (laut Kontrolle per Dünnschichtchromatographie ist das Diolgemisch vollständig umgesetzt). Es wird mit 2-normaler Natriumcarbonatlösung neutralisiert und mit gesättigter Natriumhydrogencarbonatlösung verdünnt. Mit n-Butanol wird das Produkt extrahiert und anschließend der Alkohol entfernt. Die Reinheitskontrolle erfolgt per Dünnschichtchromatographie und NMR. Ausbeute: 56,7 g (80 % d. Th.), Reinheit: 90 % (Isomerengemisch), charakteristische ¹³C-NMR Daten (DMSO-D₆):
80,7 ppm, 80,3 ppm, 73,7 ppm, 72,5 ppm, 72,3 ppm, 72,1 ppm 71,1 ppm, 69,8 ppm (beide Isomere), 34,1 ppm, 33,5 ppm, 32,0 ppm, 31,8 ppm, 31,6 ppm, 24,8 pp, 16,0 ppm.
CMC (in VE-Wasser, 20 °C): 0,2 g/l, γ_{CMC}: 33,0 mN/m (VE-Wasser, 20 °C), γ_{Paraffin} : 2,5 mN/m (Trinkwasser, 20 °C, 0,1 g/l WAS).

### Beispiel 3:

### R¹ = R³ = C₁₀H₂₁ R² = -(CH₂)₄- X = Y = SO₃Na

In einem 250 ml-Dreihalskolben mit Rührer, Thermometer, Schutzgasanschluß und Rückflußkühler werden 18,1 g 1,4-Butandiol und 3,0 Gew.-% (bezogen auf Epoxid) Al₂O₃ vorgelegt, das Gemisch bis zur Rührfähigkeit erwärmt und 83,9 g 1,2-Epoxydodekan zugegeben. Das Reaktionsgemisch wird dann auf 180 °C erwärmt und die Reaktion nach 10 Stunden (Epoxid laut GC vollständig umgesetzt) beendet. Es wird mit Natriumhydrogencarbonatlösung gewaschen. Ausbeute: 70,6 g (50 %), Reinheit laut GC > 95 %, Isomerenverhältnis: sekundäres Diol zu primärem Diol: 90 : 10.

### Sulfatierung:

Zu einem Gemisch aus 38,8 g Chlorsulfonsäure und 19,9 g Essigsäure werden 62,2 g des oben erhaltenen Diols in 200 ml Dichlormethan so zugetropft, daß die Temperatur 5 °C nicht übersteigt. Anschließend wird bei Raumtemperatur 3 Stunden gerührt (laut Kontrolle per Dünnschichtchromatographie ist das Diolgemisch vollständig umgesetzt). Es wird mit 2-normaler Natriumcarbonatlösung neutralisiert und mit gesättigter Natriumhydrogencarbonatlösung verdünnt. Mit n-Butanol wird das Produkt extrahiert und anschließend der Alkohol entfernt. Die Reinheitskontrolle erfolgt per Dünnschichtchromatographie und NMR. Ausbeute: 39,9 g (60 % d. Th.), Reinheit: 90 % (Isomerengemisch), charakteristische ¹³C-NMR Daten (DMSO-D₆):
78,2 ppm, 74,6 ppm, 74,2 ppm, 72,0 ppm, 69,8 ppm (breit), 67,3 ppm (beide Isomere), 30,8 ppm, 28,5 ppm, 28,2 ppm, 21,5 ppm, 13,1 ppm.
CMC (in VE-Wasser, 20 °C): 0,0058 g/l, γ_{CMC}: 30,0 mN/m (Trinkwasser, 20 °C), γ_{Paraffin}: 2,0 mN/m (Trinkwasser, 20 °C, 0,1 g/l WAS).

## Patentansprüche

1. Amphiphile Verbindungen der allgemeinen Formeln Ia, Ib, Ic, Id 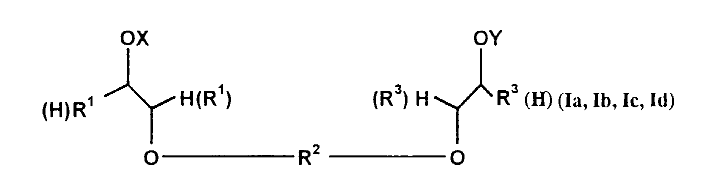
in der R¹ und R³ unabhängig voneinander einen Kohlenwasserstoffrest mit 1 bis 22 Kohlenstoffatomen, wobei R¹ und R³ in der Formel I je einmal vorkommen,
R² einen Spacer und
X und Y unabhängig voneinander die Substituenten der Formel XVI, XVII oder XVIII
-(C₂H₄O)_{α}(C₃H₆O)_{β}H (XVI)
mit α = 0 bis 50, vorzugsweise α = 10 bis 30,
β = 0 bis 60, vorzugsweise β = 20 bis 40,
und α + β = 1 bis 100, vorzugsweise α + β = 10 bis 50,
-(C₂H₄O)_{γ}(C₃H₆O)_{δ}-FR (XVII)
mit jeweils γ = 0 bis 20, vorzugsweise γ = 0 bis 8,
δ = 0 bis 20, vorzugsweise δ = 0 bis 12,
und γ + δ = 0 bis 40, vorzugsweise γ + δ = 5 bis 20,
wobei FR für einen funktionellen Rest -CH₂-COOM, -SO₃M, -P(O)(OM)₂,
-C₂H₄-SO₃M
oder
-O-C(O)-C₂H₃(SO₃M)-CO₂M'
mit M, M' = Alkali, Ammonium-, Alkanolammonium oder ½ Erdalkali steht,
-CH₂[CHO(C₂H₄O)_{ε}(C₃H₆O)_{η}H]_{z}-CH₂-O(C₂H₄O)_{µ}(C₃H₆O)_{ρ}-H (XVIII)
mit z = 3 bis 6, vorzugsweise z = 4
und ε bzw. µ = 0 bis 30, vorzugsweise ε bzw. µ = 0 bis 10,
und η bzw. ρ = 0 bis 30, vorzugsweise η bzw. ρ = 0 bis 10,
und wobei die Alkoxideinheiten statistisch oder blockweise eingebunden sind und die Reihenfolge beliebig ist, bedeuten.

2. Amphiphile Verbindungen nach Anspruch 1,
dadurch gekennzeichnet,
daß die Kohlenwasserstoffreste R¹ und R³ unverzweigt oder verzweigt, gesättigt oder ungesättigt sind und
der Spacer R² eine unverzweigte oder verzweigte Kette mit 2 bis 100 Kohlenstoffatomen, die 0 bis 20 Sauerstoff-, 0 bis 20 Stickstoff-, 0 bis 4 Schwefel- und 0 bis 3 Phosphoratome enthält und die 0 bis 20 funktionelle Seitengruppen aufweist, bedeutet.

3. Amphiphile Verbindungen nach einem der Ansprüche 1 oder 2,
dadurch gekennzeichnet,
daß die Kohlenwasserstoffreste R¹ = und R³ in den Formeln Ia, Ib, Ic und Id unabhängig voneinander 6 bis 18 Kohlenstoffatome enthalten.

4. Amphiphile Verbindungen nach einem der Ansprüche 1 bis 3,
dadurch gekennzeichnet,
daß R² einen Spacer bedeutet, der als Grundkörper eine unverzweigte oder verzweigte Alkylenkette der Formel II
-CₐH₂ₐ- (II)
mit a = 2 bis 18, vorzugsweise a = 2 bis 6,
oder eine unverzweigte oder verzweigte Alkenylenkette der Formel III
-C_{b}H_{2b}-CH=CH-C_{c}H_{2c}- (III)
mit b + c = 2 bis 16, wobei b und c jeweils größer als Null sind,
oder eine unverzweigte oder verzweigte Alkinylenkette der Formel IV
-C_{d}H_{2d}-C≡C-CₑH₂ₑ- (IV)
mit d + e = 2 bis 16, wobei d und e jeweils größer als Null sind, und wobei bei den Grundkörpern gemäß den Formeln II bis IV der Spacer an einer beliebigen Stelle der Kette unabhängig voneinander 0 bis 4 Carbonyl-, Amino- oder Acylaminogruppen aufweist,
enthält.

5. Amphiphile Verbindungen nach einem der Ansprüche 1 bis 3,
dadurch gekennzeichnet,
daß R² einen Spacer bedeutet, der aus Alicyclen gemäß der Formel V
-C_{f}H_{2f}-cyclo C₆H₁₀-C_{g}H_{2g}- (V)
mit f und g gleich unabhängig voneinander je 1 bis 6
oder gemäß der Formel VI
-3(4),8(9)-di(methylen)-tricyclo[5.2.1.0^{2.6}]decan- (VI)
besteht.

6. Amphiphile Verbindungen nach einem der Ansprüche 1 bis 3,
dadurch gekennzeichnet,
daß R² einen Spacer bedeutet, der aus unsubstituierten oder substituierten Aromaten gemäß der Formel VII
-CₕH₂ₕ-C₆R₄-(CᵢH₂ᵢ-C₆R₄)_{j₁}-C_{j₂}H_{2j₂}- (VII)
oder gemäß der Formel VIII
-CₕH₂ₕ-C₁₀R₆-CⱼH₂ⱼ- (VIII)
mit h, j, j₁ und j₂ gleich unabhängig voneinander je 0 bis 8 und i = 0 bis 8 und
mit R gleich unabhängig voneinander jeweils H oder C₁- bis C₆-Alkyl besteht.

7. Amphiphile Verbindungen nach einem der Ansprüche 1 bis 3,
dadurch gekennzeichnet,
daß der Spacer R² funktionelle Seitengruppen, insbesondere Hydroxyl-, Carbonyl-, Carboxyl-, Amino- und/oder Acylaminogruppen, trägt.

8. Amphiphile Verbindungen nach einem der Ansprüche 1 bis 3,
dadurch gekennzeichnet,
daß in dem Spacer R² je 0 bis 20, vorzugsweise 1 bis 12, Sauerstoff- und/oder Stickstoffatome, 0 bis 4 Schwefel- und 0 bis 3 Phosphoratome enthalten sind, wobei mindestens eines der Heteroatome mindestens einmal vorkommt.

9. Amphiphile Verbindungen nach Anspruch 8,
dadurch gekennzeichnet,
daß R² einen Spacer gemäß der Formel (IX)
-CₖH₂ₖ-CₓR_{y}-Z-CₓR_{y}-CₗH₂ₗ- (IX)
mit k und l gleich unabhängig voneinander je 0 bis 8, x = 6 und y = 4 oder x = 10 und y = 6 oder x = 14 und y = 8, und
Z = 0, CO, NH, NR¹, N-CH₂-CH(OX)-R¹, N-C(O)R¹, SO₂
oder gemäß der Formel IXa
-CH₂-CH(OCH₂CH(OX)-R¹)-CH₂- oder ein Isomer (IXa),
wobei R¹ einen Kohlenwasserstoffrest mit 1 bis 22 Kohlenstoffatomen bedeutet und X für einen Substituenten steht, der eine funktionelle Gruppe trägt und die weiter unten angegebene Bedeutung gemäß den Formeln XVI bis XVIII hat.

10. Amphiphile Verbindungen nach Anspruch 8,
dadurch gekennzeichnet,
daß R² einen Spacer gemäß der Formel X
(X) -CₘH₂ₘ-(OCₙH₂ₙ)ₚ-C_{q}-H_{2q}-
mit m = 1 bis 4, n = 2 bis 4, p = 1 bis 20, vorzugsweise p = 1 bis 4, und q = 0 bis 4,
wobei auch gemischte Alkoxideinheiten auftreten können und dann die Reihenfolge der Alkoxideinheiten beliebig ist,
oder 2,2'-Methylen-bis-(1,3-dioxolan-5-methylen)- oder Diacetale aus Dialdehyden und Di-, Oligo- oder Polyolen bedeutet.

11. Amphiphile Verbindungen nach Anspruch 8,
dadurch gekennzeichnet,
daß R² einen Spacer gemäß der Formel XI
-CᵣH₂ᵣ(RNCₛH₂ₛ)ₜ-CᵤH₂ᵤ- (XI)
oder gemäß der Formel XII
-[CᵣH₂ᵣ[RN-C(O)-NR]ₜ-CᵤH₂ᵤ]_{w}- (XII)
oder gemäß der Formel XIII
-[CᵣH₂ᵣ[RNC(O)CᵥH₂ᵥC(O)NR]ₜ-CᵤH₂ᵤ]_{w}- (XIII)
oder gemäß der Formel XIV
-[CᵣH₂ᵣ[RN-C(O)-CH=CH-C(O)-NR]ₜ-CᵤH₂ᵤ]_{w}- (XIV)
oder gemäß der Formel XV
-[CᵣH₂ᵣ[RNC(O)CₓR_{y}C(O)NR]ₜ-CᵤH₂ᵤ]_{w}- (XV)
mit r = 2 bis 4, s = 2 bis 4, t = 1 bis 20, vorzugsweise t = 1 bis 4,
u = 2 bis 4, v = 0 bis 12, w = 1 bis 6, x = 6 und y = 4 oder
x = 10 und y = 6 oder
x = 14 und y = 8
mit R gleich unabhängig voneinander H oder C₁- bis C₆-Alkyl,
bedeutet.

12. Amphiphile Verbindungen nach einem der Ansprüche 1 bis 11,
dadurch gekennzeichnet,
daß für ihre Herstellung aus Di-, Oligo- oder Polyolen und Monoepoxiden Alkalialkoholat oder Schichtsilikate oder Alumosilikate oder Aluminiumoxide oder Niobsäure als Katalysatoren verwendet werden.

13. Verwendung der amphiphilen Verbindungen nach einem der Ansprüche 1 bis 12 als Emulgatoren oder Demulgatoren.

14. Verwendung der amphiphilen Verbindungen nach einem der Ansprüche 1 bis 12 als Hilfsmittel bei der Metallbearbeitung, Erzgewinnung oder Oberflächenveredelung.

15. Verwendung der amphiphilen Verbindungen nach einem der Ansprüche 1 bis 12 als Textilhilfsmittel oder für das Reinigen und Waschen von Textilien.

16. Verwendung der amphiphilen Verbindungen nach einem der Ansprüche 1 bis 12 für das Reinigen von harten Oberflächen.

17. Verwendung der amphiphilen Verbindungen nach einem der Ansprüche 1 bis 12 für das Reinigen und Waschen von Haut und Haar.

## Claims

1. Amphiphilic compounds of the general formula Ia, Ib, Ic, Id 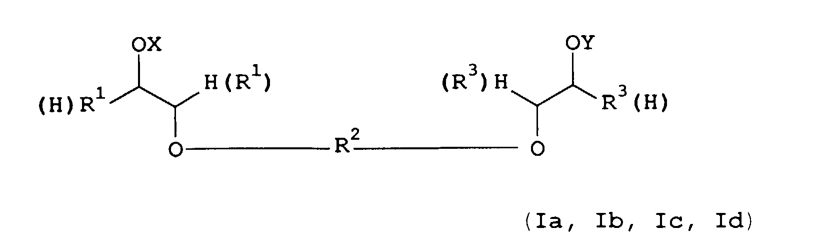
where **R**^{**1**} and **R**^{**3**}, independently of one another, are a hydrocarbon radical with 1 to 22 carbon atoms and each **R**^{**1**} and **R**^{**3**} in formula I occurs once,
**R**^{**2**} is a spacer, and
**X** and **Y**, independently of one another, are substituents of formulae XVI, XVII, or XVIII
- (C₂H₄O)_{α}(C₃H₆O)_{β} H (XVI)
where
α = 0 to 50, preferably α = 10 to 30,
β = 0 to 60, preferably β = 20 to 40,
and
α + β = 1 to 100, preferably
α + β = 10 to 50,
-(C₂H₄O)_{γ}(C₃H₆O)_{δ}-FR (XVII)
where γ = 0 to 20, preferably γ = 0 to 8,
δ = 0 to 20, preferably δ = 0 to 12,
γ + δ = 0 to 40, preferably
γ + δ = 5 to 20,
and **FR** represents a functional radical
-CH₂-COOM, -SO₃M, -P(O)(OM)₂, -C₂H₄-SO₃M, or
-O-C (O)-C₂H₃(SO₃M)-CO₂M',
wherein M, M' are equal to alkali, ammonium, alkanol ammonium, or ½ alkaline earth metal,
-CH₂[CHO(C₂H₄O)_{ε}(C₃H₆O)_{η}H]_{z}-CH₂-O(C₂H₄O)_{µ}(C₃H₆O)_{ρ}-H (XVIII)
where
**z** = 3 to 6, preferably **z** = 4,
ε or µ = 0 to 30, preferably
ε or µ = 0 to 10,
η or ρ = 0 to 30, preferably
η or ρ = 0 to 10,
and the alkoxide units are incorporated randomly or blockwise and the sequence is optional.

2. Amphiphilic compounds according to claim 1,
characterized in that
the hydrocarbon radicals **R**^{**1**} and **R**^{**3**} are unbranched or branched, saturated or unsaturated, and
the spacer **R**^{**2**} is an unbranched or branched chain with 2 to 100 carbon atoms which contains 0 to 20 oxygen, 0 to 20 nitrogen, 0 to 4 sulfur, and 0 to 3 phosphorus atoms and which has 0 to 20 functional side groups.

3. Amphiphilic compounds according to any one of claims 1 to 2,
characterized in that
the hydrocarbon radicals **R**^{**1**} and **R**^{**3**} in the formulae Ia, Ib, Ic, and Id, independently of one another, contain 6 to 18 carbon atoms.

4. Amphiphilic compounds according to any one of claims 1 to 3,
characterized in that
**R**^{**2**} is a spacer which, as a basic structure, contains an unbranched or branched alkylene chain according to formula II
-CₐH₂ₐ- (II)
where **a** = 2 to 18, preferably **a** = 2 to 6,
or an unbranched or branched alkenylene chain according to formula III
-C_{b}H_{2b}-CH=CH-C_{c}H_{2c}- (III)
where **b** + **c** = 2 to 16 and each **b** and **c** is greater than zero,
or an unbranched or branched alkynylene chain according to formula IV
-C_{d}H_{2d}-C≡C-CₑH₂ₑ- (IV)
where **d** + **e** = 2 to 16 and each **d** and **e** is greater than zero,
and where the spacer in said basic structures according to formulae II to IV, independently of one another, has 0 to 4 carbonyl, amino, or acylamino groups at any desired place in the chain.

5. Amphiphilic compounds according to any one of claims 1 to 3,
characterized in that
**R**^{**2**} represents a spacer consisting of alicycles according to formula V
-C_{f}H_{2f}-cycloC₆H₁₀-C_{g}H_{2g}- (V)
where each **f** and **g**, independently of one another, is equal to from 1 to 6,
or according to formula VI
-3(4), 8(9)-di(methylene)-tricyclo [5.2.1.0^{2.6}]decane-. (VI)

6. Amphiphilic compounds according to any one of claims 1 to 3,
characterized in that
**R**^{**2**} represents a spacer consisting of unsubstituted or substituted aromatics
according to formula VII
-CₕH₂ₕ-C₆R₄-(CᵢH₂ᵢ-C₆R₄)_{j₁}-C_{j₂}H_{2j₂}- (VII)
or according to formula VIII
-CₕH₂ₕ-C₁₀R₆-CⱼH₂ⱼ- (VIII)
where each **h, j, j**_{**1**}, and **j**_{**2**}, independently of one another, is equal to from 0 to 8, **i** is equal to from 0 to 8, and each **R**, independently of one another, is equal to H or C₁ to C₆ alkyl.

7. Amphiphilic compounds according to any one of claims 1 to 3,
characterized in that
the spacer **R**^{**2**} bears functional side groups, particularly hydroxyl, carbonyl, carboxyl, amino, and/or acylamino groups.

8. Amphiphilic compounds according to any one of claims 1 to 3,
characterized in that
each **R**^{**2**} spacer contains 0 to 20, preferably 1 to 12, oxygen and/or nitrogen atoms, 0 to 4 sulfur atoms, and 0 to 3 phosphorus atoms, with at least one of the heteroatoms occurring at least once.

9. Amphiphilic compounds according to claim 8, characterized in that
**R**^{**2**} is a spacer according to formula IX
-CₖH₂ₖ-CₓR_{y}-Z-CₓR_{y}-CₗH₂ₗ- (IX)
where each **k** and **l**, independently of one another, is equal to from 0 to 8,
**x** is equal to 6 and **y** is equal to 4, or
**x** is equal to 10 and **y** is equal to 6, or
**x** is equal to 14 and **y** is equal to 8, and
**Z** is equal to 0, CO, NH, NR¹, N-CH₂-CH(OX)-R¹, N-C(O)R¹, SO₂,
or according to formula IXa
-CH₂-CH(OCH₂CH(OX)-R¹)-CH₂- or an isomer (IXa)
where **R**^{**1**} is a hydrocarbon radical with 1 to 22 carbon atoms and **X** is a substituent which bears a functional group and has the meaning according to formulae XVI to XVIII set forth hereinbelow.

10. Amphiphilic compounds according to claim 8,
characterized in that **R**^{**2**} is
a spacer according to formula X
-CₘH₂ₘ-(OCₙH₂ₙ)ₚ-C_{q}H_{2q}- (X)
where **m** = 1 to 4, **n** = 2 to 4, **p** = 1 to 20, preferably **p** = 1 to 4, and **q** = 0 to 4, and where mixed alkoxide units may also be present and the sequence of said alkoxide units can be optional,
or 2,2'-methylene-bis-(1,3-dioxolane-5-methylene)-,
or diacetals of dialdehydes and di-, oligo-, or polyols.

11. Amphiphilic compounds according to claim 8,
characterized in that
**R**^{**2**} is a spacer
according to formula XI
-CᵣH₂ᵣ(RNCₛH₂ₛ)ₜ-CᵤH₂ᵤ- (XI)
or according to formula XII
-[CᵣH₂ᵣ[RN-C(O)-NR]ₜ-CᵤH₂ᵤ]_{w}- (XII)
or according to formula XIII
-[CᵣH₂ᵣ[RNC(O)CᵥH₂ᵥC(O)NR]ₜ-CᵤH₂ᵤ]_{w}- (XIII)
or according to formula XIV
-[CᵣH₂ᵣ[RN-C(O)-CH=CH-C(O)-NR]ₜ-CᵤH₂ᵤ]_{w}- (XIV)
or according to formula XV
-[CᵣH₂ᵣ[RNC(O)CₓR_{y}C(O)NR]ₜ-CᵤH₂ᵤ]_{w}- (XV)
where **r** = 2 to 4, **s** = 2 to 4, **t** = 1 to 20, preferably **t** = 1 to 4, **u** = 2 to 4, **v** = 0 to 12, **w** = 1 to 6, **x** = 6 and **y** = 4, or
**x** = 10 and **y** = 6, or
**x** = 14 and **y** = 8,
and where **R**, independently of one another, is equal to H or C₁ to C₆ alkyl.

12. Amphiphilic compounds according to any one of claims 1 to 11,
characterized in that
alkali alcoholate or phyllosilicates or aluminosilicates or alumina or niobic acid are used as catalysts for the preparation of said compounds from di-, oligo-, or polyols and monoepoxides.

13. Use of the amphiphilic compounds according to any one of claims 1 to 12 as emulsifiers or demulsifiers.

14. Use of the amphiphilic compounds according to any one of claims 1 to 12 as auxiliaries in metal working, ore mining, or surface finishing.

15. Use of the amphiphilic compounds according to any one of claims 1 to 12 as textile auxiliaries or for cleaning and washing textiles.

16. Use of the amphiphilic compounds according to any one of claims 1 to 12 for cleaning hard surfaces.

17. Use of the amphiphilic compounds according to any one of claims 1 to 12 for cleaning and washing skin and hair.

## Revendications

1. Composés amphiphiles de formules générales Ia, Ib, Ic, Id 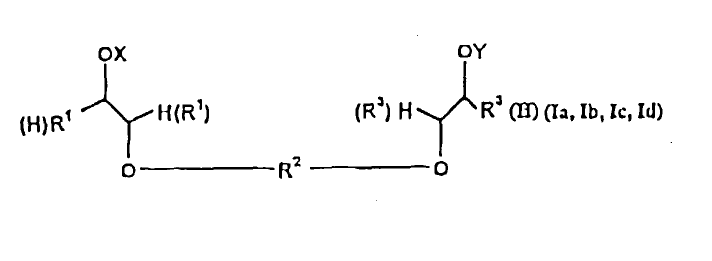
dans lesquelles R¹ et R³ désignent, indépendamment l'un de l'autre, un radical hydrocarbure ayant 1 à 22 atomes de carbone, R¹ et R³ apparaissant respectivement une fois dans la formule I,
R² est un élément d'espacement, et
X et Y désignent, indépendamment l'un de l'autre, les substituants des formules XVI, XVII ou XVIII :
-(C₂H₄O)_{α}(C₃H₆O)_{β}H (XVI)
dans laquelle α = 0 à 50, de préférence α = 10 à 30,
β = 0 à 60, de préférence β = 20 à 40, et
α + β = 1 à 100, de préférence α + β = 10 à 50,
-(C₂H₄O)_{γ}(C₃H₆O)_{δ}-FR (XVII)
dans laquelle respectivement γ = 0 à 20, de préférence
γ = 0 à 8,
δ = 0 à 20, de préférence δ = 0 à 12, et
γ + δ = 0 à 40, de préférence γ + δ = 5 à 20, et
dans laquelle FR désigne un des radicaux fonctionnels
-CH₂-COOM, -SO₃M, -P(O)(OM)₂, -C₂H₄-SO₃M ou
-O-C(O)-C₂H₃(SO₃M)-CO₂M',
avec M, M' = alcali, ammonium, alcanolammonium ou ½ alcalino-terreux,
-CH₂[CHO(C₂H₄O)_{ε}(C₃H₆O)_{η}H]_{z}-CH₂-O(C₂H₄O)_{µ}(C₃H₆O)_{ρ}-H (XVII)
dans laquelle z = 3 à 6, de préférence z = 4, et
ε ou µ = 0 à 3, de préférence ε ou µ = 0 à 10,
et η ou ρ = 0 à 30, de préférence η ou ρ = 0 à 10,
et dans laquelle les unités d'alkoxydes sont intégrées de manière statistique ou séquentielle et l'ordre est quelconque.

2. Composés amphiphiles selon la revendication 1, caractérisés en ce que les radicaux hydrocarbure R¹ et R³ sont non ramifiés ou ramifiés, saturés ou insaturés, et
l'élément d'espacement R² signifie une chaîne non ramifiée ou ramifiée ayant 2 à 100 atomes de carbone, qui contient 0 à 20 atomes d'oxygène, 0 à 20 atomes d'azote, 0 à 4 atomes de soufre et 0 à 3 atomes de phosphore et présente 0 à 20 groupes latéraux fonctionnels.

3. Composés amphiphiles selon l'une quelconque des revendications 1 et 2, caractérisés en ce que les radicaux hydrocarbure R¹ et R³ des formules Ia, Ib, Ic, et Id contiennent indépendamment l'un de l'autre 6 à 18 atomes de carbone.

4. Composés amphiphiles selon l'une quelconque des revendications 1 à 3, caractérisés en ce que R² signifie un élément d'espacement qui contient comme corps de base :
une chaîne alkylène non ramifiée ou ramifiée de formule II
-CₐH₂ₐ- (II)
dans laquelle a = 2 à 18, de préférence a = 2 à 6;
ou une chaîne alcénylène non ramifiée ou ramifiée de formule III
-C_{b}H_{2b}-CH=CH-C_{c}H_{2c}- (III)
dans laquelle b + c = 2 à 16, b et c étant respectivement supérieurs à zéro; ou
une chaîne alcinylène non ramifiée ou ramifiée
-C_{d}H_{2d}-CH≡C-CₑH₂ₑ- (IV)
dans laquelle d + e = 2 à 16, d et e étant respectivement supérieurs à zéro, et dans laquelle, dans les corps de base selon les formules II à IV, l'élément d'espacement contient en un point quelconque de la chaîne, indépendamment les uns des autres, 0 à 4 groupes carbonyle, carboxyle, amino ou acylamino.

5. Composés amphiphiles selon l'une quelconque des revendications 1 à 3, caractérisés en ce que R² signifie un élément d'espacement qui est constitué
de radicaux alicycliques de formule V
-C_{f}H_{2f}-cyclo C₆H₁₀-C_{g}H_{2g}- (V)
dans laquelle f et g sont égaux respectivement à 1 à 6, indépendamment l'un de l'autre,
ou de formule VI
-3(4),8(9)-di(méthylène)-tricyclo[5.2.1.0^{2.6}]décane (VI).

6. Composés amphiphiles selon l'une quelconque des revendications 1 à 3, caractérisés en ce que R² signifie un élément d'espacement qui est constitué d'aromatiques non substitués ou substitués de formule VII
-CₕH₂ₕ-C₆R₄-(CᵢH₂ᵢ-C₆R₄)ⱼ₁-Cⱼ₂H₂ⱼ₂- (VII)
ou de formule VIII
-CₕH₂ₕ-C₁₀R₆-CⱼH₂ⱼ- (VIII)
dans lesquelles h, j, j₁ et j₂ sont égaux, indépendamment l'un de l'autre, respectivement à 0 à 8 et i= 0 à 8 et
les R sont, indépendamment l'un de l'autre, respectivement H ou alkyle en C₁ à C₆.

7. Composés amphiphiles selon l'une quelconque des revendications 1 à 3, caractérisés en ce que l'élément d'espacement R² porte des groupes latéraux fonctionnels, en particulier des groupes hydroxyle, carbonyle, carboxyle, amino et/ou acylamino.

8. Composés amphiphiles selon l'une quelconque des revendications 1 à 3, caractérisés en ce que, dans l'élément d'espacement R², il se présente respectivement 0 à 20, de préférence 1 à 12 atomes d'oxygène et/ou atomes d'azote, 0 à 4 atomes de soufre et 0 à 3 atomes de phosphore, au moins l'un des hétéroatomes apparaissant au moins une fois.

9. Composés amphiphiles selon la revendication 8, caractérisés en ce que R² signifie un élément d'espacement de formule (X)
-CₖH₂ₖ-CₓR_{y}-Z-CₓR_{y}-CₗH₂ₗ- (IX)
dans laquelle k et 1 sont égaux, indépendamment l'un de l'autre, respectivement à 0 à 8, x = 6 et y = 4 ou x = 10 et y = 6 ou x = 14 et y = 8, et
Z = O, CO, NH, NR¹, N-CH₂-CH(OX)-R¹, N-C(O)R¹, SO₂,
ou de formule IXa
-CH₂-CH(OCH₂CH(OX)-R¹)-CH₂- ou un isomère (IXa)
dans lesquelles R¹ signifie un radical hydrocarbure ayant 1 à 22 atomes de carbone et X désigne un substituant qui porte un groupe fonctionnel et a par ailleurs la signification indiquée ci-dessous dans les formules XVI à XVIII.

10. Composés amphiphiles selon la revendication 8, caractérisés en ce que R² désigne un élément d'espacement de formule X
-CₘH₂ₘ-(OCₙH₂ₙ)ₚ-C_{q}H_{2q}- (X)
dans laquelle m = 1 à 4, n = 2 à 4, p = 1 à 20, de préférence p = 1 à 4 et q = 0 à 4,
où peuvent également apparaître des unités d'alkoxydes mixtes et où l'ordre des unités d'alkoxydes est alors quelconque,
ou le 2,2'-méthylène-bis-(1,3-dioxolane-5-méthylène)- ou des diacétals formés à partir de dialdéhydes et de di-, oligo- ou polyols.

11. Composés amphiphiles selon la revendication 8, caractérisés en ce que R² est un élément d'espacement de formule XI
-CᵣH₂ᵣ(RNCₛH₂ₛ)ₜ-CᵤH₂ᵤ- (XI)
ou de formule XII
-[CᵣH₂ᵣ[RN-C(O)-NR]ₜ-CᵤH₂ᵤ]_{w}- (XII)
ou de formule XIII
-[CᵣH₂ᵣ[RNC(O)CᵥH₂ᵥC(O)NR]ₜ-CᵤH₂ᵤ]_{w}- (XIII)
ou de formule XIV
-[CᵣH₂ᵣ[RN-C(O)-CH=CH-C(O)-NR]ₜ-CᵤH₂ᵤ]_{w}- (XIV)
ou de formule XV :
-[CᵣH₂ᵣ[RNC(O)CₓR_{y}C(O)NR]ₜ-CᵤH₂ᵤ]_{w}- (XV)
dans lesquelles r = 2 à 4, s = 2 à 4, t = 1 à 20, de
préférence t = 1 à 4, u = 2 à 4, v = 0 à 12, w = 1 à 6,
x = 6 et y = 4 ou
x = 10 et y = 6, ou
x = 14 et y = 8,
R étant, indépendamment l'un de l'autre, H ou alkyle en C₁ à C₆.

12. Composés amphiphiles selon l'une quelconque des revendications 1 à 11, caractérisés en ce que l'on utilise comme catalyseurs pour leur fabrication à partir de di-, oligo- ou polyols et de monoépoxydes, un alcoolate de métal alcalin ou un silicate stratifié ou un aluminosilicate ou un oxyde d'aluminium ou de l'acide de niobium.

13. Utilisation des composés amphiphiles selon l'une quelconque des revendications 1 à 12 comme émulsionnants ou désémulsionnants.

14. Utilisation des composés amphiphiles selon l'une quelconque des revendications 1 à 12 comme agent auxiliaire dans le traitement des métaux, la récupération des minerais ou l'ennoblissement des surfaces.

15. Utilisation des composés amphiphiles selon l'une quelconque des revendications 1 à 12 comme agent auxiliaire textile ou pour le nettoyage et le lavage de textiles.

16. Utilisation des composés amphiphiles selon l'une quelconque des revendications 1 à 12 pour le nettoyage de surfaces dures.

17. Utilisation des composés amphiphiles selon l'une quelconque des revendications 1 à 12, pour le nettoyage et le lavage de la peau et des cheveux.
